# EUROPÄISCHE PATENTSCHRIFT

(11) **EP 2 460 458 B1**
(45) Veröffentlichungstag und Bekanntmachung des Hinweises auf die Patenterteilung: **21.02.2018**
(21) Anmeldenummer: 11009419.0
(22) Anmeldetag: 29.11.2011
(51) Int. Cl.: A61B 1/005, A61B 1/018, A61B 17/04, A61B 17/00

(54) **Endoskopisches Instrumentensystem**
Endoscopic instrument system
Système d'instruments endoscopiques

(30) Priorität: 06.12.2010 DE 102010053410
(43) Veröffentlichungstag der Anmeldung: 06.06.2012
(73) Patentinhaber: Karl Storz SE & Co. KG, 78532 Tuttlingen (DE)
(72) Erfinder: Perretta, Silvana, Dr., 67000 Strasbourg (FR); Dallemagne, Bernard, Dr., 4052 Beauafys (BE); Bacher, Uwe, 78532 Tuttlingen (DE); Zahler, Sabine, 85560 Ebersberg (DE); Wagner, Sebastian, 75015 Bretten (DE)
(74) Vertreter: Fugmann, Winfried

(56) Entgegenhaltungen:
- EP-A1- 1 987 789
- DE-A1-102008 053 880
- US-A1- 2002 111 534
- US-A1- 2005 096 694

## Beschreibung

Die vorliegende Erfindung betrifft ein endoskopisches Instrumentensystem insbesondere zur Durchführung endoskopischer Eingriffe über einen natürlichen Zugangsweg nach dem Oberbegriff von Anspruch 1.

Endoskopische Operationstechniken haben sich für eine Vielzahl chirurgischer Eingriffe durchgesetzt. Ein wesentlicher Vorteil endoskopisch durchgeführter gegenüber konventionellen offenen Operationen ist, dass die Notwendigkeit eines großen Einschnitts in der Haut und im darunterliegenden Gewebe zur Zugänglichmachung des Operationsgebiets entfällt, wodurch eine erhebliche Belastung des Patienten vermieden und die Dauer der stationären Behandlung verringert werden kann. Stattdessen werden beispielsweise bei endoskopisch durchgeführten Eingriffen im Bauchraum Endoskope bzw. endoskopische Instrumente durch eine oder mehrere kürzere Inzisionen zum Operationsgebiet geführt, wo etwa durch Gasinsufflation ein Hohlraum geschaffen werden kann, innerhalb dessen die für die Durchführung des Eingriffs notwendigen chirurgischen Manipulationen durchgeführt werden können. Nach Durchführung der Manipulationen werden die Endoskope bzw. endoskopischen Instrumente aus dem Bauchraum entnommen und die durchgetrennten Gewebeschichten geschlossen, beispielsweise durch chirurgische Nähte. Auch wenn die Inzisionen und daher die notwendigen Nähte bei derartigen laparoskopischen Eingriffen relativ kurz sind, können dabei dennoch unter Umständen Narben zurückbleiben.

In EP 1 987 789 A1 ist ein Endoskopsystem offenbart, das ein flexibles Endoskop mit einem langen Einführungsteil und ein medizinisches Instrument umfasst. Das medizinische Instrument beinhaltet einen in einem distalen Bereich angeordneten Behandlungsteil und einen langen Schaft, der in einen Kanal in dem Einführungsteil eingeführt wird. Das Endoskopsystem umfasst ferner einen Bedienungsapparat zur Bedienung des medizinischen Instruments, einen ersten Antriebsapparat zum motorischen Betreiben des Behandlungsteils und einen zweiten Antriebsapparat, der den Schaft des medizinischen Instruments motorisch vor- und rückwärts bewegt. Der Schaft des medizinischen Instruments weist einen starren Teil mit vorbestimmter Flexibilität und einen flexiblen Teil mit einer demgegenüber höheren Flexibilität auf. Der starre Teil ragt bei der Benutzung des Behandlungsteils aus der Öffnung eines Instrumenteneinführteils des Endoskops heraus. Dies erlaubt dem Chirurgen, den starren Teil zu ergreifen, um zur Veränderung der Orientierung des Behandlungsteils den Schaft zu drehen. Eine in proximale Richtung übertragene Torsionsspannung wird durch den flexiblen Teil absorbiert und dadurch ein Brechen des Schafts vermieden.

Aus US 2002/01115334 A1 sind ein Endoskop und ein endoskopisches Instrument zur Behandlung der gastroösophagealen Refluxkrankheit bekannt, wobei eine Haltevorrichtung zum Halten von Gewebe und eine Nadel zum Durchstechen des Gewebes und zum Durchführen eines Fadens vorgesehen sind.

Um die Belastung des Patienten bei chirurgischen Eingriffen im Bauchraum weiter zu verringern, ist ein unter der Bezeichnung NOTES (Natural Orifice Transluminal Endoscopic Surgery) bekanntes Operationsverfahren entwickelt worden, bei dem die benötigten Endoskope bzw. endoskopischen Instrumente durch natürliche Körperöffnungen, beispielsweise durch den Rachen, die Speiseröhre und den Magen, in den Körper eingeführt werden (s. www.wikipedia.de, Stichwort "NOTES", vom 06.12.2010). Über einen kleinen Schnitt in der Magenwand kann das eigentliche Operationsgebiet im Bauchraum, beispielsweise die Gallenblase, erreicht werden. Da die Magenwand weniger Schmerzrezeptoren aufweist als die normale Haut, ist hierbei ein geringerer Einsatz von Anästhetika notwendig, wodurch die Erholungszeit des Patienten weiter verkürzt wird. Ferner kann die Wirkung der Magensäure zur Verhinderung von Infektionen ausgenutzt werden. Schließlich können sichtbare Narben ganz oder, wenn zusätzlich ein endoskopischer Zugang durch die Körperoberfläche notwendig ist, zumindest teilweise vermieden werden. Endoskope zur Durchführung von Operationen nach dem NOTES-Verfahren sind beispielsweise aus den Offenlegungsschriften US 2008/0269562 A1 und US 2009/0054733 A1 bekannt, welche hiermit diesbezüglich durch Bezugnahme in die vorliegende Anmeldung aufgenommen werden.

Nach Durchführung der Manipulationen innerhalb des Bauchraums und Entnahme der Endoskope bzw. endoskopischen Instrumente aus dem Operationsgebiet soll die Inzision in dem Organ, das den natürlichen Zugang darstellt, beispielsweise in der Magenwand, wieder verschlossen werden. Auch bei dem chirurgischen Eingriff selbst kann es erforderlich sein, Gewebeöffnungen zu verschließen bzw. Geweberänder miteinander zu verbinden. Hierfür sind unterschiedliche Techniken bekannt, beispielsweise Clips, Gewebeanker oder Klammern. Diese sind jedoch nicht nur mit einem erheblichen instrumentellen Aufwand verbunden, sondern weisen auch beispielsweise den Nachteil auf, dass nach der Operation zunächst ein Fremdkörper verbleibt, der aufgrund seiner mechanischen Eigenschaften die normale Funktion der Magenwand behindern kann. Ferner sind die Einsatzmöglichkeiten derartiger Verschlussmittel begrenzt. Der Wiederverschluss der Eintrittspforte ist daher bei der NOTES-Methode bisher nicht befriedigend gelöst. Häufig wird die Inzision in der Wand des Zugangsorgans deshalb gar nicht verschlossen.

Es wäre somit wünschenswert, ein endoskopisches Instrumentensystem zur Durchführung von Operationen durch einen natürlichen Zugangsweg (NOTES) zur Verfügung zu haben, das hinsichtlich des Verschlusses der Inzision in der Wand des Zugangsorgans verbessert ist. Ein solcher Verschluss könnte insbesondere durch eine chirurgische Naht in der Art erfolgen, die zum Verschluss einer Inzision in der Körperoberfläche oder in oberflächennahem Weichgewebe verwendet wird. Die Möglichkeiten zur Ausführung einer Naht im Rahmen einer derartigen Operation sind jedoch bisher sehr begrenzt.

Zum Erstellen derartiger Nähte sind insbesondere bei Operationen, die mit starren Endoskopen durchgeführt werden können, Nadelhalter zum Greifen von chirurgischen Nadeln bekannt. Diese können gerade, gerade mit einer gekrümmten Spitze, gebogen oder teilkreisförmig ausgebildet sein und werden zum Ausführen eines Stiches und zum Durchführen eines chirurgischen Fadens durch die beiden miteinander zu verbindenden Geweberänder näherungsweise auf einer Kreisbahn geführt. Hierfür kann je nach Art und Dicke des Gewebes sowie nach Art der Nadel ein erhebliches Drehmoment erforderlich sein.

Im Rahmen des NOTES-Verfahren werden flexible endoskopische Instrumente eingesetzt. Zur Erhöhung der Drehsteifigkeit des Schafts kann dieser beispielsweise als Drahtspirale ausgebildet oder mit einer anderen Art von Verstärkung versehen sein. Dennoch ist bei bekannten endoskopischen Instrumenten zum Einsatz in flexiblen Endoskopen für die Durchführung einer Operation nach der NOTES-Methode das übertragbare Drehmoment für eine entsprechende Handhabung zum Durchstechen von Gewebe mit einer chirurgischen Nadel aufgrund der Flexibilität des Instruments häufig nicht ausreichend. Auch für andere Manipulationen, beispielsweise für das Durchtrennen von härterem Gewebe, ist die mit derartigen Instrumenten ausübbare Kraft bzw. das ausübbare Drehmoment nicht immer ausreichend für eine rasche und sichere Durchführung der Operation.

Es ist die Aufgabe der vorliegenden Erfindung, ein endoskopisches Instrumentensystem zu schaffen, insbesondere für die Durchführung einer endoskopischen Operation durch einen natürlichen Zugang, das die oben genannten Nachteile nicht aufweist und wodurch insbesondere das Erstellen einer chirurgischen Naht zum Verschließen der Eingangspforte in dem Zugangsorgan verbessert wird.

Diese Aufgabe wird durch ein endoskopisches Instrumentensystem gemäß Anspruch 1 gelöst.

Ein erfindungsgemäßes endoskopisches Instrumentensystem, das insbesondere für die Durchführung endoskopischer Eingriffe über einen natürlichen Zugangsweg geeignet ist, umfasst ein flexibles Endoskop mit einem Endoskophandgriff und einem langerstreckten Endoskopschaft, der in ein Hohlorgan, das den natürlichen Zugangsweg bildet, einführbar ist. Der Endoskopschaft kann ferner dafür geeignet sein, durch eine Inzision in der Wand des Hohlorgans weiter in das Körperinnere, beispielsweise in den Bauchraum, eingeführt zu werden. Der Endoskopschaft umfasst einen steuerbaren, insbesondere abwinkelbaren Endabschnitt. Ferner weist der Endoskopschaft mindestens einen Instrumentenkanal auf, der im Inneren des Endoskopschafts angeordnet ist und von einem proximalen, d.h. benutzernahen, Endbereich zu einem distalen, d.h. benutzerfernen Endbereich des Endoskopschafts durchgehend ausgeführt ist.

Das flexible Endoskop kann weiterhin eine Optik zur Erfassung und Weiterleitung eines endoskopischen Bildes vom distalen zum proximalen Endbereich des Endoskops aufweisen, die insbesondere ein Objektiv und einen Lichtleiter, etwa ein geordnetes Glasfaserbündel, umfassen kann. Am proximalen Ende des Endoskops, insbesondere am Endoskophandgriff, kann ein Kameraanschluss vorgesehen sein bzw. mit dem proximalen Ende des Endoskops kann ein Kameramodul zur Aufnahme des weitergeleiteten endoskopischen Bildes verbunden sein. Es kann aber auch ein elektronischer Bildaufnehmer im distalen Endbereich des Endoskopschafts angeordnet sein. Ferner kann das Endoskop eine Lichtquelle bzw. einen Lichtanschluss und eine Beleuchtungsoptik zur Beleuchtung des körperinneren Hohlraums bzw. des Operationsgebiets umfassen. Weiterhin können ein oder mehrere weitere Instrumentenkanäle vorhanden sein sowie insbesondere am Endoskophandgriff bzw. am Kameramodul angeordnete Spül- und Sauganschlüsse sowie Bedieneinrichtungen zum Steuern des steuerbaren Endabschnitts des Endoskopschafts. Ein für die Durchführung endoskopischer Eingriffe über einen natürlichen Zugangsweg gemäß dem NOTES-Verfahren geeignetes Endoskop kann auch über am distalen Ende des Endoskopschafts angeordnete Abdeckklappen verfügen, die zum Einführen des Endoskops über den natürlichen Zugangsweg sowie durch eine Inzision im Zugangsorgan einen Obturator bilden können und/oder zum Steuern durch den Endoskopschaft geführter endoskopischer Instrumente dienen können.

Das endoskopische Instrumentensystem umfasst weiterhin ein endoskopisches Instrument, das eine Handhabe, einen Instrumentenschaft und ein Werkzeug umfasst. Das Werkzeug ist durch ein innerhalb des Instrumentenschafts angeordnetes und mit der Handhabe verbundenes Zug- bzw. Druckelement betätigbar. Das Zug- bzw. Druckelement ist in bevorzugter Weise als Zugelement zum Ausüben einer Zugkraft ausgebildet, kann aber auch als Druckelement zum Ausüben einer Druckkraft oder sowohl zum Ausüben einer Zug- wie einer Druckkraft ausgebildet sein. Das endoskopische Instrument ist zur Ausführung von Manipulationen in dem körperinneren Hohlraum, in den das Endoskop eingeführt werden kann, in den Instrumentenkanal einführbar. Die Länge des Instrumentenschafts kann insbesondere derart bemessen sein, dass nach dem Einführen des endoskopischen Instruments in den Instrumentenkanal des Endoskops das Werkzeug ausreichend über das distale Ende des Endoskopschafts hinaus geführt werden kann, um die für einen Eingriff notwendigen Manipulationen ausführen zu können. Die Handhabe kann dann am proximalen Endbereich des Endoskopschafts oder am Endoskophandgriff anliegen oder zur Erleichterung der Bedienung auch einen gewissen Abstand zum proximalen Ende des Endoskopschafts bzw. zum Endoskophandgriff aufweisen.

Der Instrumentenschaft weist ein durchgehendes biegsames Rohr auf, innerhalb dessen das Zug- bzw. Druckelement zum Betätigen des Werkzeugs angeordnet ist. Insbesondere kann der Instrumentenschaft allein aus dem durchgehenden biegsamen Rohr bestehen, der Instrumentenschaft kann aber beispielsweise auch ein zusätzliches Außenrohr oder eine außenseitige Beschichtung aufweisen. Der Instrumentenschaft ist daher nach Art eines starren endoskopischen Instruments aufgebaut und insbesondere nicht segmentiert und nicht durch einen gewundenen Draht, ein durch Fenster oder Einschnitte geschwächtes Rohr oder ein Gewebe gebildet. Das durchgehende Rohr ist aufgrund seiner Ausführung, insbesondere aufgrund eines geringen Außendurchmessers bzw. aufgrund der Verwendung von elastischem hochfestem Material, ausreichend biegsam, um in einem flexiblen Endoskop verwendet zu werden. Das durchgehende biegsame Rohr ist sowohl mit der Handhabe als auch mit dem Werkzeug bezüglich einer Längsrichtung des Rohrs drehfest verbunden. Die Handhabe ermöglicht daher nicht nur ein Betätigen des Werkzeugs mit Hilfe des Zug- bzw. Druckelements und ein Verschieben des Werkzeugs in Längsrichtung des Instrumentenschafts, d.h. in Längsrichtung des Endoskopschafts, sondern auch ein Drehen des Werkzeugs um eine Längsachse des Rohrs, d.h. um eine Längsachse des Instrumentenschafts bzw. des Endoskopschafts. Ein erfindungsgemäßes endoskopisches Instrumentensystem kann weitere, ähnlich oder unterschiedlich ausgebildete Instrumente zum Durchführen eines chirurgischen Eingriffs umfassen.

Dadurch, dass der Instrumentenschaft ein durchgehendes Rohr aufweist, wird eine besonders hohe Drehsteifigkeit des Instrumentenschafts erreicht, so dass eine besonders genau steuerbare Drehbewegung des Werkzeugs um eine Längsachse des Instrumentenschafts und die Übertragung eines besonders hohen Drehmoments von der Handhabe auf das Werkzeug ermöglicht wird. Dies erleichtert die Durchführung chirurgischer Manipulationen für eine Vielzahl von Anwendungen, beispielsweise zum Schneiden von hartem Gewebe. Dadurch, dass das durchgehende Rohr biegsam ist, wird die Kombination mit einem flexiblen Endoskop und damit insbesondere die Anwendung im Rahmen des NOTES-Verfahrens ermöglicht.

Gemäß einer bevorzugten Ausführungsform der Erfindung ist das Werkzeug zum Halten einer chirurgischen Nadel ausgebildet. Üblicherweise sind chirurgische Nadeln, die zum Verschließen von Inzisionen bei endoskopischen Eingriffen vorgesehen sind, halbrund oder teilkreisförmig ausgebildet. Eine derartige Nadel ist beispielsweise aus der Druckschrift DE 10 2009 007 722 A1 bekannt, welche diesbezüglich durch Bezugnahme in die vorliegende Anmeldung aufgenommen wird. Zum Einstechen der Spitze einer solchen Nadel wird diese üblicherweise mit einem Nadelhalter in einem mittleren oder hinteren, d.h. von der Spitze der Nadel entfernten, Bereich derart gegriffen, dass die Ebene des Teilkreises der Nadel näherungsweise senkrecht zu einer Längsachse des Nadelhalters steht. Durch eine Drehung des Nadelhalters um die Längsachse wird sodann die Spitze der Nadel in das Gewebe eingestochen. Dadurch, dass mit einem erfindungsgemäßen endoskopischen Instrumentensystem die Übertragung eines besonders hohen Drehmoments auf das Werkzeug und damit auf die gehaltene Nadel möglich ist, kann durch ein flexibles Endoskop eine chirurgische Naht in der in der Laparoskopie üblichen Weise, d.h. mit üblichem endoskopischem Nahtmaterial und einer insbesondere halbrunden Nadel, ausgeführt werden. Hierdurch können bei der Durchführung endoskopischer Eingriffe über einen natürlichen Zugangsweg gemäß dem NOTES-Verfahren insbesondere die Nachteile der Verwendung von Clips oder Klammern vermieden werden, und es ist in einer weitaus größeren Zahl von Fällen überhaupt möglich, die in die Wand des Zugangsorgans eingebrachte Inzision wieder zu verschließen.

Insbesondere kann das Werkzeug in vorteilhafter Weise zum Fassen und zum Halten einer chirurgischen Nadel ausgebildet sein. Ein derartiges, als Nadelhalter ausgebildetes endoskopisches Instrument ermöglicht es insbesondere, eine halbrunde chirurgische Nadel zu fassen, zu halten, durch eine Rotation um eine Längsachse des Instruments die Nadel in Gewebe einzustechen und teilweise durch das Gewebe durchzuführen, die Nadel loszulassen, in einem bereits durch das Gewebe geführten Bereich zu erneut zu fassen und mit einem in die Nadel eingehängten Faden vollständig durch das Gewebe durchzuziehen. Die Ausbildung des endoskopischen Instruments mit einem Instrumentenschaft, der als durchgehendes Rohr ausgeführt ist, ermöglicht die Übertragung besonders hoher Kräfte zur Betätigung des Werkzeugs, wodurch die Nadel mit einer hohen Kraft gegriffen werden und ein Wegdrehen der Nadel sicher vermieden werden kann. Hierdurch wird eine besonders einfache und sichere Bedienung des Nadelhalters in der üblichen Weise zur Erstellung einer endoskopischen Naht ermöglicht.

Um eine chirurgische Nadel zu fassen und zu halten, kann das Werkzeug in an sich bekannter Weise zwei gegeneinander wirkende Maulteile aufweisen. Davon kann eines beweglich, beispielsweise um eine zur Längsachse des Instrumentenschafts quer stehende Achse schwenkbar sein und das andere relativ zum distalen Ende des Instrumentenschafts feststehend. Es können aber auch beide Maulteile relativ zum distalen Endes des Instrumentenschafts beweglich sein, beispielsweise symmetrisch um eine gemeinsame, quer zur Längsachse des Instrumentenschafts stehende Achse gegeneinander schwenkbar. Ein derartiges Öffnen und Schließen der Maulteile kann über das Zug- bzw. Druckelement durch die Handhabe gesteuert werden. Zum sicheren Fassen und Halten der Nadel können die Maulteile an ihren gegeneinander wirkenden Flächen beispielsweise Querrippen oder -rillen aufweisen. Hierdurch wird eine besonders einfache Bedienung und eine sichere Ausführung der Naht ermöglicht. In vorteilhafter Weise kann das Werkzeug mit den zwei gegeneinander wirkenden Maulteilen auch oder stattdessen zum Fassen und Halten von Gewebe ausgebildet sein.

Erfindungsgemäß weist zumindest ein erster Abschnitt des biegsamen Rohrs einen minimalen Biegeradius auf, der kleiner oder gleich einem minimalen Biegeradius des Endoskopschafts in einem ersten Abschnitt des Endoskopschafts ist, wobei der erste Abschnitt des biegsamen Rohrs derart angeordnet ist, dass dieser nach dem Einführen des endoskopischen Instruments in das Endoskop zur Ausführung der Manipulationen in dem körperinneren

Hohlraum in dem ersten Abschnitt des Endoskopschafts zu liegen kommt. Unter dem minimalen Biegeradius wird hier derjenige Biegeradius verstanden, bis zu dem ein Biegen des Rohrs bzw. des Endoskopschafts möglich ist, ohne dass eine plastische Verformung oder, bei Zulassung einer plastischen Verformung, zumindest ohne dass ein Bruch des Materials eintritt und ohne dass beispielsweise das Rohr einknickt, wodurch der Hohlraum innerhalb des Rohrs, in dem das Zug- bzw. Druckelement verläuft, blockiert oder erheblich eingeengt würde. Da insbesondere bei mehrfach verwendbaren Instrumenten ein vielfaches Biegen in unterschiedlichen Richtungen erfolgt, soll bei einem Biegen mit einem Radius, der den minimalen Biegeradius nicht unterschreitet, möglichst auch keine Materialermüdung auftreten. Der minimale Biegeradius des Instrumentenschafts wird im Wesentlichen durch das biegsame Rohr bestimmt und wird hier daher mit dessen Biegeradius gleichgesetzt. Der minimale Biegeradius des Endoskopschafts kann beispielsweise durch eine Grundstruktur der Schafts, die etwa in an sich bekannter Weise aus gelenkig miteinander verbundenen Elementen bestehen kann, bestimmt sein. In besonders vorteilhafter Weise ist der minimale Biegeradius des Rohrs um so viel kleiner als der minimale Biegeradius des betreffenden Abschnitts des Endoskopschafts, dass bei einer exzentrischen Anordnung des Instrumentenkanals relativ zu einer Längsachse des Endoskopschafts auch bei einem Biegen in unterschiedlichen Richtungen der minimale Biegeradius des Rohrs nicht unterschritten wird.

Dadurch, dass der minimale Biegeradius des ersten Abschnitts des biegsamen Rohrs kleiner oder gleich dem minimalen Biegeradius des Endoskopschafts in demjenigen Abschnitt des Endoskopschafts ist, in dem der erste Abschnitt des biegsamen Rohrs bei der Ausführung der Manipulationen angeordnet ist, ist sichergestellt, dass bei einer Biegung des Endoskops, die aufgrund der Krümmung des Zugangswegs eintreten kann oder die durch die Steuerung eines steuerbaren Abschnitts des Endoskopschafts erzwungen wird, das biegsame Rohr nicht stärker gekrümmt wird, als ohne einen Bruch oder eine andere Beschädigung möglich ist. Hierdurch wird eine erhöhte Sicherheit der Bedienung des Instrumentariums erreicht.

Erfindungsgemäß weist der erste Abschnitt des biegsamen Rohrs einen kleineren minimalen Biegeradius auf als ein zweiter Abschnitt des biegsamen Rohrs, der beispielsweise der übrige Teil des biegsamen Rohrs sein kann. Da eine Ausführung mit einem kleineren minimalen Biegeradius in der Regel mit einer geringeren Drehsteifigkeit und/oder einem geringeren übertragbaren Drehmoment verbunden ist, kann hierdurch erreicht werden, dass in denjenigen Abschnitten, in denen kein kleiner Biegeradius des Rohrs notwendig ist, dieses zur Erzielung einer hohen Drehsteifigkeit bzw. eines hohen übertragbaren Drehmoments ausgebildet ist. Der minimale Biegeradius des Instrumentenschafts ist somit abschnittsweise dem jeweiligen minimalen Biegeradius des Endoskops angepasst. Hierdurch kann ferner der Gefahr einer Buckelbildung des Instrumentenschafts innerhalb des Instrumentenkanals, der in der Regel einen größeren Innendurchmesser aufweist als dem Außendurchmesser des Instrumentenschafts entspricht, beim Ausüben einer in Längsrichtung gerichteten Kraft vorgebeugt werden.

So kann beispielsweise das biegsame Rohr in einem Abschnitt, der im eingesetzten Zustand des endoskopischen Instruments einem Abschnitt des Endoskopschafts entspricht, in dem dieser einen großen Biegeradius aufweist bzw. nur geringen Biegungen ausgesetzt ist, derart ausgebildet sein, dass das Rohr einen großen Biegeradius aufweist, während es in Abschnitten, die im eingesetzten Zustand stärker auf Biegung beansprucht werden, mit einem geringeren Biegeradius ausgebildet sein kann. In besonders vorteilhafter Weise erstreckt sich der Abschnitt des kleineren minimalen Biegeradius soweit über den entsprechenden Abschnitt des Endoskops hinaus, dass auch bei einer Hin- und Herbewegung des endoskopischen Instruments, die für die Durchführung der Manipulationen erforderlich ist, keine Biegebelastung über den jeweiligen minimalen Biegeradius hinaus erfolgen kann.

Gemäß einer besonders bevorzugten Ausführungsform der Erfindung ist der erste Abschnitt des biegsamen Rohrs ein distaler Endabschnitt des biegsamen Rohrs und der erste Abschnitt des Endoskopschafts der steuerbare Endabschnitt des Endoskopschafts. In der Regel weist der steuerbare Endabschnitt des Endoskopschafts einen kleineren Biegeradius auf als der übrige Teil des Endoskopschafts und wird beim Einsatz auch häufiger auf Biegung, noch dazu in unterschiedliche Richtungen, beansprucht. Dadurch, dass der distale Endabschnitt des biegsamen Rohrs einen Biegeradius aufweist, der kleiner ist als der des steuerbaren Endabschnitts des Endoskopschafts und der auch kleiner sein kann als der des übrigen Teils des biegsamen Rohrs, wird eine ungehinderte Benutzung der Steuerung des distalen Endabschnitts des Endoskopschafts ermöglicht, um beispielsweise durch Abwinkelung in unterschiedlichen Richtungen unterschiedliche Bereiche in einer Körperhöhle zu erreichen.

Insbesondere kann der distale Endabschnitt des Instrumentenschafts eine Länge aufweisen, die der Länge des steuerbaren Endabschnitts des Endoskopschafts entspricht zuzüglich eines Vorschubbetrags, um den das Werkzeug zur Ausführung der Manipulationen zumindest vorübergehend über das distale Ende des Endoskopschafts hinausgeschoben werden muss. Hierdurch wird auch eine ungehinderte Nutzung der Bewegung in Längsrichtung ermöglicht.

In bevorzugter Weise ist auch die Biegesteifigkeit des biegsamen Rohrs im ersten Abschnitt, der dem steuerbaren Endabschnitt des Endoskopschafts entspricht, geringer als in einem zweiten Abschnitt. Hierdurch wird bei einer Biegung des steuerbaren Endabschnitts die Reibung, die einer Drehung des Instrumentenschafts um seine Längsachse entgegensteht, verringert.

In bevorzugter Weise umfasst das biegsame Rohr einen dritten Abschnitt, der einen kleineren minimalen Biegeradius und/oder eine geringere Biegesteifigkeit aufweist als der zweite Abschnitt, wobei der dritte Abschnitt des biegsamen Rohrs bei der Ausführung der Manipulationen in dem körperinneren Hohlraum im Bereich eines proximalen Endabschnitts des Endoskopschafts angeordnet ist. Bei vielen flexiblen Endoskopen ist ein proximaler Eingang des Instrumentenkanals abgewinkelt zur Längsachse des Schafts im jeweiligen Bereich angeordnet. Der erste und der zweite Abschnitt müssen nur bei der Einführung des endoskopischen Instruments in den Instrumentenkanal bzw. beim Herausziehen bei einer Benutzung des endoskopischen Instruments die Abwinkelung überwinden, während der dritte Abschnitt bei der Benutzung mehrfach im Bereich der Abwinkelung bewegt wird. Durch den geringeren Biegeradius bzw. die geringere Biegesteifigkeit in diesem Abschnitt kann daher erreicht werden, dass eine mehrfache Längsbewegung und/oder eine Drehbewegung des Instrumentenschafts im Bereich dieser Abwinkelung ermöglicht bzw. erleichtert wird.

Der dritte Abschnitt des biegsamen Rohrs kann insbesondere ein proximal angeordneter Endabschnitt des Rohrs sein, an dem die Handhabe ansetzt. Es kann aber in proximaler Richtung noch ein weiterer Abschnitt des Rohrs bis zur Handhabe folgen.

In bevorzugter Weise umfasst das Endoskop ein proximales gerades Führungsrohr, in das der Instrumentenkanal des Endoskopschafts einmündet. Das biegsame Rohr kann insbesondere in diesem Fall einen proximal des dritten Abschnitts angeordneten vierten Abschnitt aufweisen, der einen größeren minimalen Biegeradius und/oder eine höhere Biegesteifigkeit aufweist als der dritte Abschnitt des biegsamen Rohrs. Da das Führungsrohr gerade ist, wird der beim Einsatz in diesem befindliche Abschnitt des Instrumentenschafts nicht auf Biegung belastet, so dass eine Ausführung mit einem größeren Biegeradius bzw. einer größeren Biegesteifigkeit zur Erzielung einer höheren Drehsteifigkeit bzw. der Übertragung eines höheren Drehmoments vorteilhaft ist. Ferner kann hierdurch ein Verkanten beim Bewegen des endoskopischen Instruments in Längsrichtung vermieden werden.

Insbesondere können der minimale Biegeradius und/oder die Biegesteifigkeit auch größer sein als im zweiten Abschnitt des Rohrs, weil das Führungsrohr einen größeren Innendurchmesser haben kann als ein Instrumentenkanal innerhalb des Endoskopschafts, da das Führungsrohr nicht in den Körper eingeführt wird und daher nicht den hierdurch gegebenen Beschränkungen unterliegt.

Die Handhabe kann auch im eingesetzten Zustand des endoskopischen Instruments noch einen Abstand von einem proximalen Eingang des Instrumentenkanals bzw. des Führungsrohrs aufweisen. In diesem Fall kann eine höhere Biegesteifigkeit zur Erleichterung der Bedienung und zur Verhinderung eines Abknickens des Instrumentenschafts dienen.

In bevorzugter Weise ist das biegsame Rohr aus Rohrabschnitten aus unterschiedlichen Materialien und/oder mit unterschiedlichen Außendurchmessern zusammengesetzt. Sowohl durch unterschiedliche Materialien als auch durch unterschiedliche Außendurchmesser des Rohrs können unterschiedliche minimale Biegeradien erreicht werden. Ferner kann auch die Wandstärke unterschiedlicher Abschnitte des Rohrs unterschiedlich sein, um unterschiedliche Biegesteifigkeiten zu erzielen.

In vorteilhafter Weise können die Rohrabschnitte durch Schweißen, insbesondere Laserschweißen, und/oder Hartlöten und/oder Kleben miteinander verbunden sein. Insbesondere kann der Innendurchmesser eines Rohrabschnitts mit größerem Außendurchmesser derart gewählt sein, dass dieser auf einen Rohrabschnitt mit einem geringeren Außendurchmesser aufgeschoben werden und mit diesem sodann durch Schweißen bzw. Hartlöten verbunden werden kann. Hierdurch wird eine ausreichend haltbare, gegen temperatur- und chemische Einflüsse beständige und rotationssteife Verbindung erzielt. Ferner kann sowohl beim Schweißen als auch beim Hartlöten die entstehende Hohlkehle beim Verbinden von Rohren unterschiedlicher Außendurchmesser aufgefüllt werden. Alternativ oder zusätzlich kann die Oberfläche im Verbindungsbereich glatt geschliffen werden, um die Reibung zu vermindern und ein Hängenbleiben beim Bewegen des Instrumentenschafts zu verhindern. Ein Kleben durch Auffüllen des Ringspalts zwischen den unterschiedlich großen Rohrabschnitten mit Klebstoff hat den Vorteil, dass Kerbspannungen und mögliche Materialveränderungen aufgrund der thermischen Beanspruchung beim Schweißen bzw. Löten verringert oder vermieden werden können.

Gemäß einer bevorzugten Ausführungsform weist das biegsame Rohr und daher der Instrumentenschaft abschnittsweise unterschiedliche minimale Biegeradien auf, wobei das biegsame Rohr in einem Abschnitt mit kleinerem minimalem Biegeradius einen kleineren Außendurchmesser aufweist als in einem Abschnitt mit einem größeren minimalen Biegeradius. Alternativ oder zusätzlich hierzu kann das biegsame Rohr in einem Abschnitt mit kleinerem minimalem Biegeradius aus einem Material mit einer größeren Verformbarkeit bzw. bei gleichem Elastizitätsmodul mit einer höheren Zugfestigkeit bestehen als in einem Abschnitt mit einem größeren minimalen Biegeradius. Da der minimale Biegeradius im Wesentlichen vom Außendurchmesser und den Eigenschaften des Materials abhängt, kann hierdurch eine vorteilhafte Gestaltung des Instrumentenschafts mit unterschiedlichen Biegeradien erreicht werden. Unterschiedliche Biegesteifigkeiten können neben dem Elastizitätsmodul des Materials auch durch Variation der Wandstärke des Rohrs bestimmt werden. Das Zug- bzw. Druckelement wird bevorzugt derart flexibel ausgeführt, beispielsweise als Zugseil oder als Zug- bzw. Druckstange aus einem elastischen Metall, dass der minimale Biegeradius des Zug- bzw. Druckelements stets geringer oder zumindest nicht wesentlich größer als der des Rohrs ist. Je nach Ausführung kann das Zug- bzw. Druckelement auch zur Biegesteifigkeit des Instrumentenschafts beitragen.

In bevorzugter Weise ist der minimale Biegeradius des Rohrs der minimale elastische Biegeradius, d.h. ein solcher Biegeradius, bis zu dem das Rohr ohne eine plastische Verformung gebogen werden kann. Hierdurch wird gewährleistet, dass die Reibung bei einer Bewegung des endoskopischen Instruments innerhalb eines mit dem Endoskop gekrümmten Instrumentenkanals, insbesondere der bei einer Drehung um seine Längsachse zu überwindende Widerstand, möglichst gering ist und unabhängig von vorherigen Bewegungen und von der Richtung einer Biegung des Endoskopschafts. Ferner kann durch Vermeiden einer plastischen Verformung mit größerer Sicherheit einer Ermüdung des Materials vorgebeugt werden.

Geeignete Materialien, aus denen das biegsame Rohr zumindest abschnittsweise bestehen kann, sind beispielsweise Federstahl, Phynox sowie superelastische Metalle, beispielsweise Nitinol. Das biegsame Rohr kann aber auch aus Edelstahl oder aus anderen metallischen oder auch aus keramischen Materialien, beispielsweise aus Zirkonoxid-Keramik, hergestellt werden. Eine hohe Verformbarkeit und damit ein geringer Biegeradius, bei Inkaufnahme plastischer Verformungen, kann auch durch Verwendung von Monel erzielt werden.

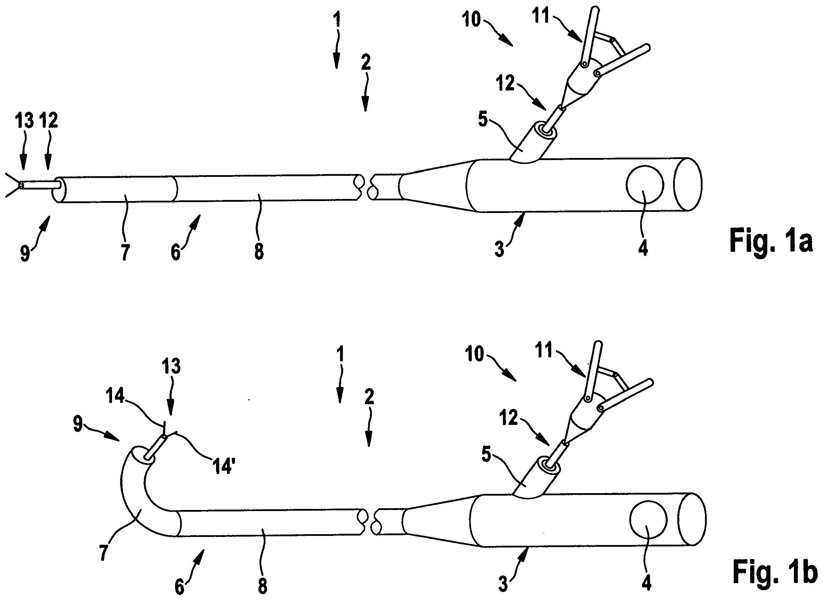

Weitere Aspekte der Erfindung ergeben sich aus der nachfolgenden Beschreibung bevorzugter Ausführungsbeispiele und der beigefügten Zeichnung. Es zeigen:
Fig. 1a und 1b ein erfindungsgemäßes endoskopisches Instrumentensystem;
Fig. 2 ein erfindungsgemäßes endoskopisches Instrument gemäß einer ersten Ausführungsform;
Fig. 3 ein erfindungsgemäßes endoskopisches Instrument gemäß einer zweiten Ausführungsform;
Fig. 4 ein biegsames Rohr eines erfindungsgemäßen Instruments.
Ein erfindungsgemäßes endoskopisches Instrumentensystem 1 umfasst ein flexibles Endoskop 2 und ein endoskopisches Instrument 10, wobei das Endoskop 2 in Fig. 1a in einem gestreckten Zustand dargestellt ist. Das Endoskop 2 umfasst einen Endoskophandgriff 3, der ein symbolisch dargestelltes Bedienelement 4 zum Steuern einer Abwinkelung und ein Führungsrohr 5 aufweist, sowie einen Endoskopschaft 6. Der Endoskopschaft 6 umfasst einen distalen Endabschnitt 7 und einen proximalen Abschnitt 8, der die gesamte übrige Länge des Endoskopschafts 6 einnehmen und deutlich länger als der distale Endabschnitt 7 sein kann. Innerhalb des Endoskopschafts 6 erstreckt sich ein Instrumentenkanal, der sich in den Endoskophandgriff 3 fortsetzt und dort in das abgewinkelt ansetzende Führungsrohr 5 mündet. Das endoskopische Instrument 10 besteht aus einer Handhabe 11, einem langerstreckten Instrumentenschaft 12 und einem Werkzeug 13. Der Instrumentenschaft 12 wird durch ein biegsames Rohr gebildet, innerhalb dessen ein Zugelement zwischen Handhabe 11 und Werkzeug 13 verläuft.

Fig. 1b zeigt das endoskopische Instrumentensystem der Fig. 1a in einem abgewinkelten Zustand. Hierfür können über das Bedienelement innerhalb des Endoskopschafts 6 verlaufende Seilzüge betätigt werden, die eine Abwinkelung des distalen Endabschnitts 7 des Endoskopschafts 6 bewirken. Insbesondere der distale Endabschnitt 7 kann eine Stützstruktur aus gelenkig miteinander verbundenen Schaftsegmenten aufweisen, die die Seilzüge führen und deren Beweglichkeit einen minimalen Biegeradius des distalen Endabschnitts 7 bestimmt. Der distale Endabschnitt 7 oder der gesamte Endoskopschaft 6 können von einer flexiblen Hülle, die beispielsweise ein Schlauch aus einem Kunststoffmaterial sein kann, umgeben sein.

In dem in Fig. 1a und 1b gezeigten Zustand ist der Instrumentenschaft 12 mit dem an dessen distalem Ende angeordneten Werkzeug 13 in das Führungsrohr 5 und den daran anschließenden Instrumentenkanal eingeführt. Durch Betätigung der am proximalen Ende des Endoskops angeordneten Handhabe 11, die durch einen in Fig. 1a lediglich symbolisch dargestellten Mechanismus auf das innerhalb des Instrumentenschafts 12 bewegliche Zugelement einwirkt, kann das Werkzeug 13 betätigt werden. Zum Einführen des endoskopischen Instruments 10 in den Instrumentenkanal kann das Werkzeug 13 in eine geschlossene Stellung gebracht werden (nicht dargestellt).

Wie in Fig. 1b durch die geöffneten Maulteile 14, 14' angedeutet, kann das Werkzeug 13 auch im abgewinkelten Zustand des distalen Endabschnitts 7 des Endoskopschafts 6 betätigt werden, so dass beispielsweise bei einer Ausgestaltung als Nadelhalter eine Nadel gegriffen und gehalten werden kann. Der minimale Biegeradius des Instrumentenschafts 12 ist in demjenigen Abschnitt, der innerhalb des abwinkelbaren distalen Endabschnitts 7 angeordnet ist, geringer oder gleich dem minimalen Biegeradius des distalen Endabschnitts 7, um eine uneingeschränkte Verwendung des endoskopischen Instruments 10 zu ermöglichen. Der im eingeführten Zustand innerhalb des proximalen Abschnitts 8 des Endoskopschafts 6, der gegenüber dem distalen Endabschnitt 7 weniger flexibel sein muss, angeordnete Abschnitt des Instrumentenschafts 12 kann demgegenüber mit einem größeren minimalen Biegeradius ausgeführt sein. Dies ist hinsichtlich der Drehsteifigkeit und der Übertragung eines Drehmoments durch Drehung der Handhabe 11 um die Längsachse des proximalen Endbereichs des Instrumentenschafts 12 vorteilhaft, um eine exakte und mit einem hohen Drehmoment erfolgende Drehung des Werkzeugs 13 um die Längsachse des distalen Endbereichs des Instrumentenschafts 12 zu bewirken. Hierfür sind sowohl die Handhabe 11 als auch das Werkzeug 13 bezüglich der jeweiligen Längsachse drehfest mit dem Rohr, das den Instrumentenschaft 12 bildet, verbunden.

Ferner kann die Biegesteifigkeit des Instrumentenschafts 12 in dem im distalen Endabschnitt 7 des Endoskopschafts 6 angeordneten Abschnitt gegenüber dem im proximalen Abschnitt 8 befindlichen Abschnitt verringert sein, um die bei einer Bewegung des Instrumentenschafts 12 im Instrumentenkanal auftretende Reibung zu verringern. Zur Verringerung der Reibung kann ferner ein in den Instrumentenkanal mündender Spülanschluss am Führungsrohr 5 vorgesehen sein (nicht dargestellt).

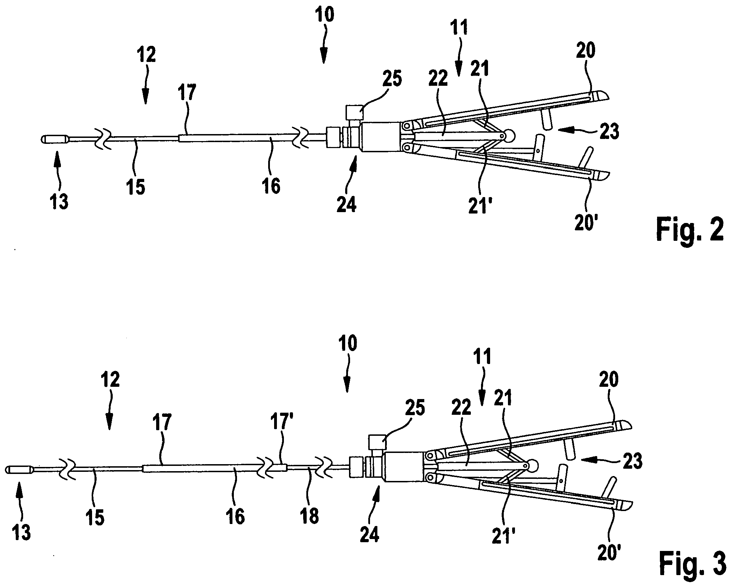

Ein erfindungsgemäßes endoskopisches Instrument 10 ist als Ganzes in Fig. 2 dargestellt. Der Instrumentenschaft 12 umfasst einen ersten, distalen Abschnitt 15, dessen Länge näherungsweise der des steuerbaren Endabschnitts 7 entspricht, und einen zweiten Abschnitt 16, der in dem in Fig. 2 dargestellten Ausführungsbeispiel die gesamte übrige Länge des Instrumentenschafts bis zur Handhabe 11 einnehmen kann. Wie in Fig. 2 symbolisch dargestellt, weist der erste Abschnitt 15 einen geringeren Außendurchmesser auf als der zweite Abschnitt 16. Sind beide Abschnitte aus dem gleichen Material ausgeführt, so ist der minimale Biegeradius des ersten Abschnitts 15 kleiner als der des zweiten Abschnitts 16. In einem Verbindungsbereich 17 sind die beiden Schaftabschnitte 15, 16 miteinander verbunden.

Die Handhabe 11 umfasst zwei an einem Grundelement 24 unter einem flachen Winkel gegen die Verlängerung der Längsachse des Instrumentenschafts 12 geneigt angeordnete Handgriffe 20, 20', die zur Betätigung des Werkzeugs 13 gegen eine Federkraft zusammengedrückt werden können, so dass über Hebel 21, 21' und ein mit dem Zugelement verbundenes Übertragungselement 22 eine Zugkraft auf das Zugelement ausgeübt und dieses relativ zum Instrumentenschaft 12 verschoben werden kann. Durch Betätigen der Handgriffe 20, 20' können daher die beiden Maulteile 14, 14' des Werkzeugs 13 geöffnet oder geschlossen werden. Um beispielsweise eine gegriffene Nadel sicherer halten zu können, ist eine lösbare Raste 23 zum Fixieren der Handgriffe 20, 20' in einer geschlossenen oder teilweise geschlossenen Stellung vorgesehen. Ferner kann ein Spülanschluss, der in Fig. 2 durch eine Abdeckkappe 25 abgedeckt ist, vorhanden sein.

Das in dem Instrumentenschafts 12 geführte Zugelement kann innerhalb des distalen und des proximalen Endbereichs des Instrumentenschafts 12 durch Dichtungen gegen diesen abgedichtet sein (nicht dargestellt). Sowohl im distalen wie im proximalen Endbereich können hierfür ein oder mehrere O-Ringe vorgesehen sein, die dichtend zwischen Instrumentenschaft 12 und Zugelement angeordnet sind; insbesondere im distalen Endbereich kann hierfür aber auch beispielsweise ein Ziehharmonika-artig faltbares verschweißtes Rohr vorgesehen sein. Eine Prüfung der Dichtigkeit des endoskopischen Instruments 10 kann dann beispielsweise durch ein im proximalen Endbereich des Instrumentenschafts 12, jedoch distalseitig der proximalen Dichtung angeordnetes Belüftungsventil ermöglicht werden.

In der in Fig. 3 gezeigten Variante umfasst der Instrumentenschaft 12 einen dritten Abschnitt 18, der proximal des zweiten Abschnitts 16 angeordnet ist und im eingeführten Zustand des endoskopischen Instruments 10 im Bereich der Mündung des Instrumentenkanals in das abgewinkelte Führungsrohr 5, die sich innerhalb des Endoskophandgriffs 3 befindet, zu liegen kommt. Der dritte Abschnitt 18 weist einen gegenüber dem zweiten Abschnitt 16 geringeren Außendurchmesser auf und ist über einen Verbindungsbereich 17' mit dem zweiten Abschnitt 16 verbunden. Dadurch, dass der dritte Abschnitt 18 einen verringerten Außendurchmesser aufweist, wird in dem Bereich, in dem der Instrumentenschaft 12 die Krümmung vom Führungsrohr 5 in den Instrumentenkanal überwinden muss, ein geringerer minimaler Biegeradius und eine geringere Biegesteifigkeit erzielt, was die Handhabung des endoskopischen Instruments 10 verbessert. Dadurch, dass nur die distalen und proximalen Abschnitte 15, 18 einen verringerten Durchmesser aufweisen, wird die Übertragung eines maximalen Drehmoments und eine maximale Drehsteifigkeit gewährleistet. Im Übrigen ist das in Fig. 3 dargestellte Ausführungsbeispiel wie das in Fig. 2 gezeigte ausgebildet.

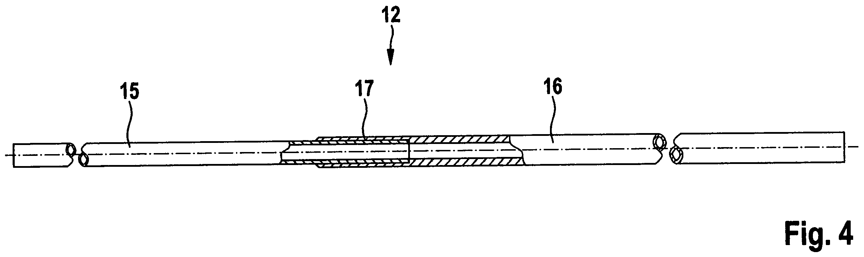

In Fig. 4 ist ein biegsames Rohr eines erfindungsgemäßen Instruments in teilweise aufgeschnittener Längsschnittdarstellung gezeigt. Der erste Abschnitt 15 weist einen gegenüber dem zweiten Abschnitt 16 verringerten Außendurchmesser und deshalb einen verringerten minimalen Biegeradius auf. Der erste Abschnitt 15 kann beispielsweise durch ein Phynoxrohr und der zweite Abschnitt durch ein Edelstahlrohr gebildet werden. Im Verbindungsbereich 17 sind beide Rohrabschnitte übereinander geschoben und miteinander verbunden, beispielsweise durch Laserschweißen oder Hartlöten. In dem nicht über das Rohr des ersten Abschnitts 15 geschobenen Teil weist das Rohr des zweiten Abschnitts 16 den gleichen Innendurchmesser auf wie das des ersten Abschnitts, um eine reibungsarme Bewegung des in dem Rohr geführten, nicht dargestellten Zugelements zu gewährleisten. Das gesamte durchgehende biegsame Rohr kann auch aus mehreren Abschnitten, beispielsweise aus zwei Phynoxrohren und zwei Edelstahlrohren ggf. unterschiedlicher Durchmesser bestehen.

Gemäß vorteilhafter Ausführungsbeispiele der Erfindung beträgt die Länge des Endoskopschafts 6 je nach Anwendung bevorzugt mehr als ca. 400 mm, insbesondere für NOTES-Operationen durch die Magenwand mehr als ca. 1000 mm. Der Instrumentenkanal weist einen Innendurchmesser von beispielsweise ca. 1,7 bis 4,2 mm (entsprechend etwa 5 bis 13 Charrière) auf. Der minimale Biegeradius des Endoskopschafts 6 liegt im Bereich des distalen Endabschnitts 7 bei ca. 25 mm bis ca. 60 mm, bevorzugt 30 mm bis 40 mm, und ist in den übrigen Abschnitten des Endoskopschafts größer, beispielsweise ca. 100 mm.

Länge, Außendurchmesser und minimaler Biegeradius eines gemeinsam mit dem Endoskop einzusetzenden endoskopischen Instruments sind dem jeweiligen Endoskop angepasst. Der Instrumentenschaft 12 besteht beispielsweise aus einem durchgehenden biegsamen Rohr mit einem Außendurchmesser von 1,2 mm bzw. 1,4 mm. Bei Verwendung von Phynox oder Federstahl wird ein minimaler elastischer Biegeradius ca. 60 mm, bei Verwendung von Nitinol von ca. 30 bis 40 mm erreicht; bei einem Außendurchmesser des Rohrs von 0,8 mm beträgt bei Verwendung von Federstahl der minimale Biegeradius ca. 40 mm. Bei Verwendung in dem flexiblen Endoskop kann jeweils eine geringfügige plastische Verformung in Kauf genommen werden. Die Wandstärke des biegsamen Rohrs beträgt ca. 0,05 mm bis 0,5 mm und die Länge bevorzugt mehr als 400 mm, insbesondere mehr als 1000 mm.

Bei einer endoskopischen Operation im Bauchraum nach dem NOTES-Verfahren wird das Endoskop 2 unter endoskopischer Sicht durch einen natürlichen Zugangsweg, beispielsweise durch Rachen, Speiseröhre und Magen, eingeführt. Dabei wird der distale Endabschnitt 7 zur Erleichterung der Einführung entsprechend gesteuert. Das endoskopische Instrument 10, das beispielsweise als Nadelhalter ausgeführt sein kann, ist zu diesem Zeitpunkt noch nicht in das Endoskop eingeführt. Mit Hilfe eines durch einen Instrumentenkanal eingeführten Schneidwerkzeugs wird eine Inzision in der Magenwand vorgenommen. Durch diese Inzision wird sodann das Endoskop 2 in den Bauchraum eingeführt. Durch Gasinsufflation kann ein Hohlraum geschaffen werden, in dem die eigentliche endoskopische Operation stattfindet, wofür weitere Instrumente eingeführt werden können, beispielsweise Greifzangen, Biopsiezangen, Scheren, Elektroden, Fangkörbchen usw. Es kann aber auch lediglich ein diagnostischer Eingriff stattfinden, für den keine weiteren chirurgischen Manipulationen notwendig sind. Durch Abwinkelung des distalen Endabschnitts 7 des Endoskopschafts 6 kann die Blick- und Arbeitsrichtung entsprechend den jeweiligen Erfordernissen gewählt werden. Dabei kann eine Beleuchtung über eine im Endoskop enthaltene Beleuchtungsoptik sowie eine Aufnahme des Bildes durch eine im distalen oder im proximalen Bereich des Endoskops angeordnete CCD-Kamera erfolgen.

Nach Beendigung des eigentlichen Eingriffs wird das Endoskop mit ggf. eingesetzten Instrumenten aus dem Bauchraum durch die Inzision in den Magen zurückgezogen. Zum Verschluss der Inzision wird nun ein als Nadelhalter ausgeführtes endoskopisches Instrument eingeführt, bevorzugt bei möglichst gestrecktem Endoskopschaft 6. Soweit erforderlich, wird der distale Endabschnitt 7 des Endoskopschafts 6 abgewinkelt, sobald das Werkzeug 13 mit den Maulteilen 14, 14' aus dem distalen Ende 9 des Endoskopschafts heraustritt. Mit den Maulteilen 14, 14' des Nadelhalters wird eine ebenfalls eingeführte halbrunde Nadel im hinteren Teil in der Nähe des in die Nadel eingehängten Fadens gegriffen. Die Spitze der Nadel steht nun ca. 10 bis 15 mm seitlich gegenüber den Maulteilen 14, 14' vor. Hiermit wird nun in die miteinander zu verbindenden Ränder der Inzision durch Rotation des Instrumentenschafts 12 eingestochen, bis die Spitze der Nadel wieder aus dem Gewebe heraustritt. Zum Fassen und Platzieren der Nadel sowie zum Drehen des Instrumentenschafts 12 wird die Handhabe 11 entsprechend betätigt. Das notwendige Drehmoment zum Bewegen der Nadel wird durch eine Drehung der Handhabe 11 erzeugt und über das durchgehende Rohr, das den Instrumentenschaft 12 bildet, auf die Maulteile 14, 14', die die Nadel halten, übertragen. Zum Ausüben der erforderlichen Kräfte auf das Gewebe kann im vorliegenden Fall das Endoskop als Widerlager dienen, insbesondere dessen steuerbarer distaler Endabschnitt 7, der durch den Steuerungsmechanismus ausreichend steif gehalten werden kann und sich relativ großflächig abstützt.

Die Nadelspitze wird mit einem weiteren Instrument, beispielsweise einer Greifzange, gegriffen. Sodann wird der hintere Teil der Nadel mit dem Nadelhalter losgelassen und die Nadelspitze mit dem Nadelhalter gegriffen. Die Nadel wird dann aus dem Gewebe herausgezogen. Im nächsten Schritt wird der Faden durch das Gewebe gezogen, bis vom losen Ende nur noch ausreichend Faden zurückbleibt, um einen Knoten bilden zu können. Der Vorgang wird ggf. wiederholt, und es wird in üblicher Weise endoskopisch ein Knoten gebildet. Sodann wird das Fadenende abgeschnitten, an dem die Nadel hängt. Hierfür können ein oder mehrere weitere Instrumente erforderlich sein, die ggf. durch weitere Instrumentenkanäle eingeführt werden können. In entsprechender Weise kann sodann der nächste Knoten gebildet werden.

Nach dem Erstellen der Naht wird das Endoskop mit dem endoskopischen Instrument aus dem natürlichen Zugangsweg heraus gezogen.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Endoskopisches Instrumentensystem
- 2: Endoskop
- 3: Endoskophandgriff
- 4: Bedienelement
- 5: Führungsrohr
- 6: Endoskopschaft
- 7: Distaler Abschnitt
- 8: Proximaler Abschnitt
- 9: Distales Ende
- 10: Endoskopisches Instrument
- 11: Handhabe
- 12: Instrumentenschaft
- 13: Werkzeug
- 14, 14': Maulteil
- 15: Erster Abschnitt
- 16: Zweiter Abschnitt
- 17,: 17'Verbindungsbereich
- 18: Dritter Abschnitt
- 20, 20': Handgriff
- 21, 21': Hebel
- 22: Übertragungselement
- 23: Raste
- 24: Grundelement
- 25: Abdeckkappe

## Patentansprüche

1. Endoskopisches Instrumentensystem, insbesondere zur Durchführung endoskopischer Eingriffe über einen natürlichen Zugangsweg, umfassend ein flexibles Endoskop (2) mit einem Endoskophandgriff (3) und einem in einen körperinneren Hohlraum einführbaren, langerstreckten Endoskopschaft (6), der einen steuerbaren Endabschnitt umfasst, wobei der Endoskopschaft (6) mindestens einen Instrumentenkanal aufweist, und ein endoskopisches Instrument (10) mit einer Handhabe (11), einem langerstreckten Instrumentenschaft (12) und einem Werkzeug (13), das durch ein innerhalb des Instrumentenschafts (12) angeordnetes und mit der Handhabe (11) und mit dem Werkzeug (13) verbundenes Zug- bzw. Druckelement betätigbar ist, wobei das endoskopische Instrument (10) zur Ausführung von Manipulationen in dem körperinneren Hohlraum in den Instrumentenkanal einführbar ist, wobei der Instrumentenschaft (12) ein durchgehendes biegsames Rohr aufweist, das mit der Handhabe (11) und dem Werkzeug (13) bezüglich einer Längsrichtung des Rohrs drehfest verbunden ist, wobei die Handhabe (11) ein Drehen des Werkzeugs (13) um eine Längsachse des Rohrs ermöglicht, **dadurch gekennzeichnet, dass** zumindest ein erster Abschnitt des biegsamen Rohrs einen minimalen Biegeradius aufweist, der kleiner oder gleich einem minimalen Biegeradius des Endoskopschafts (6) in einem ersten Abschnitt des Endoskopschafts ist, wobei der erste Abschnitt des biegsamen Rohrs bei der Ausführung der Manipulationen in dem körperinneren Hohlraum in dem ersten Abschnitt des Endoskopschafts (6) angeordnet ist, und dass der erste Abschnitt des biegsamen Rohrs einen kleineren minimalen Biegeradius aufweist als ein zweiter Abschnitt, wobei der zweite Abschnitt im eingesetzten Zustand des endoskopischen Instruments einem Abschnitt des Endoskopschafts entspricht, in dem dieser einen größeren minimalen Biegeradius aufweist als im ersten Abschnitt, wobei ein minimaler Biegeradius des Instrumentenschafts abschnittsweise einem jeweiligen minimalen Biegeradius des Endoskops angepasst ist.

2. Instrumentensystem nach Anspruch 1, **dadurch gekennzeichnet, dass** das Werkzeug (13) zum Halten einer chirurgischen Nadel ausgebildet ist.

3. Instrumentensystem nach Anspruch 1 oder 2, **dadurch gekennzeichnet, dass** das Werkzeug (13) zwei gegeneinander wirkende Maulteile (14, 14') aufweist.

4. Instrumentensystem nach einem der vorhergehenden Ansprüche, **dadurch gekennzeichnet, dass** der erste Abschnitt des biegsamen Rohrs ein distaler Endabschnitt des biegsamen Rohrs ist und der erste Abschnitt des Endoskopschafts (6) der steuerbare Endabschnitt des Endoskopschafts ist.

5. Instrumentensystem nach dem vorhergehenden Anspruch, **dadurch gekennzeichnet, dass** das biegsame Rohr einen dritten Abschnitt umfasst, der einen kleineren minimalen Biegeradius und/oder eine geringere Biegesteifigkeit aufweist als der zweite Abschnitt, wobei der dritte Abschnitt des biegsamen Rohrs bei der Ausführung der Manipulationen in dem körperinneren Hohlraum im Bereich eines proximalen Endabschnitts des Endoskopschafts (6) angeordnet ist.

6. Instrumentensystem nach dem vorhergehenden Anspruch, **dadurch gekennzeichnet, dass** das Endoskop (2) ein gerades Führungsrohr (5) umfasst, in das der Instrumentenkanal des Endoskopschafts (6) mündet, und dass das biegsame Rohr einen vierten Abschnitt aufweist, der proximal des dritten Abschnitts angeordnet ist und der einen größeren minimalen Biegeradius und/oder eine höhere Biegesteifigkeit aufweist als der dritte Abschnitt des biegsamen Rohrs.

7. Instrumentensystem nach einem der vorhergehenden Ansprüche, **dadurch gekennzeichnet, dass** das biegsame Rohr aus Rohrabschnitten aus unterschiedlichen Materialien und/oder mit unterschiedlichen Außendurchmessern zusammengesetzt ist.

8. Instrumentensystem nach dem vorhergehenden Anspruch, **dadurch gekennzeichnet, dass** die Rohrabschnitte durch Schweißen und/oder Hartlöten und/oder Kleben miteinander verbunden sind.

9. Instrumentensystem nach einem der vorhergehenden Ansprüche, **dadurch gekennzeichnet, dass** das biegsame Rohr abschnittsweise unterschiedliche minimale Biegeradien aufweist, wobei das biegsame Rohr in einem Abschnitt mit kleinerem minimalem Biegeradius einen kleineren Außendurchmesser aufweist als in einem Abschnitt mit einem größeren minimalen Biegeradius.

10. Instrumentensystem nach einem der vorhergehenden Ansprüche, **dadurch gekennzeichnet, dass** das biegsame Rohr abschnittsweise unterschiedliche minimale Biegeradien aufweist, wobei das biegsame Rohr in einem Abschnitt mit kleinerem minimalem Biegeradius aus einem Material mit einer größeren Verformbarkeit bzw. bei einem gleichen Elastizitätsmodul einer höheren Zugfestigkeit besteht als in einem Abschnitt mit einem größeren minimalen Biegeradius.

11. Instrumentensystem nach einem der vorhergehenden Ansprüche, **dadurch gekennzeichnet, dass** der minimale Biegeradius der minimale elastische Biegeradius ist.

12. Instrumentensystem nach einem der vorhergehenden Ansprüche, **dadurch gekennzeichnet, dass** das biegsame Rohr zumindest abschnittsweise aus Federstahl, Phynox und/oder einem superelastischen Metall hergestellt ist.

## Claims

1. Endoscopic instrument system, in particular for carrying out endoscopic interventions via a natural access route, comprising a flexible endoscope (2) with an endoscope grip (3) and with an elongate endoscope shaft (6) which is insertable into a hollow space in a body and has a controllable end portion, wherein the endoscope shaft (6) has at least one instrument channel, and comprising an endoscopic instrument (10) with a handle (11), an elongate instrument shaft (12) and a tool (13) that can be actuated by a pulling and/or pushing element which is arranged inside the instrument shaft (12) and which is connected to the handle (11) and to the tool (13), wherein the endoscopic instrument (10) is insertable into the instrument channel in order to perform manipulations in the hollow space in the body, wherein the instrument shaft (12) has a continuous flexible tube which is connected to the handle (11) and to the tool (13) in a rotationally fixed manner with respect to a longitudinal direction of the tube, wherein the handle (11) permits a rotation of the tool (13) about a longitudinal axis of the tube, **characterized in that** at least a first portion of the flexible tube has a minimum bending radius which is smaller than or equal to a minimum bending radius of the endoscope shaft (6) in a first portion of the endoscope shaft, wherein the first portion of the flexible tube is arranged in the first portion of the endoscope shaft (6) when the manipulations are being performed in the hollow space in the body, and **in that** the first portion of the flexible tube has a smaller minimum bending radius than a second portion, wherein the second portion, in the inserted state of the endoscopic instrument, corresponds to a portion of the endoscope shaft in which the latter has a greater minimum bending radius than in the first portion, wherein a minimum bending radius of the instrument shaft is adapted in parts to a respective minimum bending radius of the endoscope.

2. Instrument system according to Claim 1, **characterized in that** the tool (13) is designed to hold a surgical needle.

3. Instrument system according to Claim 1 or 2, **characterized in that** the tool (13) has two jaw members (14, 14') acting in an opposing manner with respect to each other.

4. Instrument system according to one of the preceding claims, **characterized in that** the first portion of the flexible tube is a distal end portion of the flexible tube, and the first portion of the endoscope shaft (6) is the controllable end portion of the endoscope shaft.

5. Instrument system according to the preceding claim, **characterized in that** the flexible tube comprises a third portion which has a smaller minimum bending radius and/or a lower flexural strength than the second portion, wherein the third portion of the flexible tube is arranged in the region of a proximal end portion of the endoscope shaft (6) when the manipulations are being performed in the hollow space in the body.

6. Instrument system according to the preceding claim, **characterized in that** the endoscope (2) comprises a straight guide tube (5) into which the instrument channel of the endoscope shaft (6) leads, and **in that** the flexible tube has a fourth portion which is arranged proximally with respect to the third portion and which has a greater minimum bending radius and/or a higher flexural strength than the third portion of the flexible tube.

7. Instrument system according to one of the preceding claims, **characterized in that** the flexible tube is composed of tube portions made of different materials and/or with different external diameters.

8. Instrument system according to the preceding claim, **characterized in that** the tube portions are connected to each other by welding and/or hard soldering and/or adhesive bonding.

9. Instrument system according to one of the preceding claims, **characterized in that** the flexible tube has different minimum bending radii in parts, wherein the flexible tube has a smaller external diameter in a portion with a smaller minimum bending radius than in a portion with a greater minimum bending radius.

10. Instrument system according to one of the preceding claims, **characterized in that** the flexible tube has different minimum bending radii in parts, wherein the flexible tube, in a portion with a smaller minimum bending radius, is made of a material with a greater deformability or, if the modulus of elasticity is the same, a higher tensile strength than in a portion with a greater minimum bending radius.

11. Instrument system according to one of the preceding claims, **characterized in that** the minimum bending radius is the minimum elastic bending radius.

12. Instrument system according to one of the preceding claims, **characterized in that** the flexible tube is produced at least in part from spring steel, Phynox and/or a superelastic metal.

## Revendications

1. Système d'instrument endoscopique, en particulier pour réaliser des interventions endoscopiques par le biais d'une voie d'accès naturelle, comprenant un endoscope flexible (2) avec une poignée d'endoscope (3) et une tige d'endoscope (6) allongée pouvant être introduite dans une cavité intérieure du corps, qui comprend une portion d'extrémité commandable, la tige d'endoscope (6) présentant au moins un canal d'instrument et un instrument endoscopique (10) avec une manette (11), une tige d'instrument allongée (12) et un outil (13) qui peut être actionné par un élément de traction et/ou de pression disposé à l'intérieur de la tige d'instrument (12) et connecté à la manette (11) et à l'outil (13), l'instrument endoscopique (10) pouvant être introduit dans la cavité à l'intérieur du corps dans le canal d'instrument pour réaliser des manipulations, la tige d'instrument (12) présentant un tube flexible continu qui est connecté de manière solidaire en rotation à la manette (11) et à l'outil (13) par rapport à une direction longitudinale du tube, la manette (11) permettant une rotation de l'outil (13) autour d'un axe longitudinal du tube, **caractérisé en ce qu'**au moins une première portion du tube flexible présente un rayon de courbure minimal qui est inférieur ou égal à un rayon de courbure minimal de la tige d'endoscope (6) dans une première portion de la tige d'endoscope, la première portion du tube flexible, lors de la réalisation des manipulations dans la cavité intérieure du corps, étant disposée dans la première portion de la tige d'endoscope (6), et **en ce que** la première portion du tube flexible présente un rayon de courbure minimal plus petit qu'une deuxième portion, la deuxième portion, dans l'état inséré de l'instrument endoscopique, correspondant à une portion de la tige d'endoscope dans laquelle celle-ci présente un rayon de courbure minimal supérieur à celui dans la première portion, un rayon de courbure minimal de la tige d'instrument étant adapté en partie à un rayon de courbure minimal respectif de l'endoscope.

2. Système d'instrument selon la revendication 1, **caractérisé en ce que** l'outil (13) est réalisé pour retenir une aiguille chirurgicale.

3. Système d'instrument selon la revendication 1 ou 2, **caractérisé en ce que** l'outil (13) présente deux parties de mâchoires (14, 14') agissant l'une contre l'autre.

4. Système d'instrument selon l'une quelconque des revendications précédentes, **caractérisé en ce que** la première portion du tube flexible est une portion d'extrémité distale du tube flexible et la première portion de la tige d'endoscope (6) est la portion d'extrémité commandable de la tige d'endoscope.

5. Système d'instrument selon la revendication précédente, **caractérisé en ce que** le tube flexible comprend une troisième portion qui présente un rayon de courbure minimal plus petit que et/ou une rigidité en flexion inférieure à la deuxième portion, la troisième portion du tube flexible étant disposée dans la région d'une portion d'extrémité proximale de la tige d'endoscope (6) lors de la réalisation des manipulations dans la cavité intérieure du corps.

6. Système d'instrument selon la revendication précédente, **caractérisé en ce que** l'endoscope (2) comprend un tube de guidage droit (5) dans lequel débouche le canal d'instrument de la tige d'endoscope (6), et **en ce que** le tube flexible présente une quatrième portion qui est disposée à proximité de la troisième portion et qui présente un rayon de courbure minimal plus grand et/ou de plus grande rigidité en flexion que la troisième portion du tube flexible.

7. Système d'instrument selon l'une quelconque des revendications précédentes, **caractérisé en ce que** le tube flexible est constitué de portions de tube constituées de matériaux différents et/ou avec des diamètres extérieurs différents.

8. Système d'instrument selon la revendication précédente, **caractérisé en ce que** les portions de tube sont connectées les unes aux autres par soudage et/ou par brasage dur et/ou par collage.

9. Système d'instrument selon l'une quelconque des revendications précédentes, **caractérisé en ce que** le tube flexible présente en partie des rayons de courbure minimaux différents, le tube flexible, dans une portion ayant un rayon de courbure minimal plus petit, présentant un diamètre extérieur plus petit que dans une portion ayant un rayon de courbure minimal plus grand.

10. Système d'instrument selon l'une quelconque des revendications précédentes, **caractérisé en ce que** le tube flexible présente en partie des rayons de courbure minimaux différents, le tube flexible, dans une portion ayant un rayon de courbure minimal plus petit, étant constitué d'un matériau ayant une plus grande aptitude à la déformation ou, pour un même module d'élasticité, une résistance à la traction supérieure que dans une portion ayant un plus grand rayon de courbure minimal.

11. Système d'instrument selon l'une quelconque des revendications précédentes, **caractérisé en ce que** le rayon de courbure minimal est le rayon de courbure élastique minimal.

12. Système d'instrument selon l'une quelconque des revendications précédentes, **caractérisé en ce que** le tube flexible est fabriqué au moins en partie en acier à ressort, en Phynox et/ou en un métal superélastique.
